(19)

**Europäisches Patentamt**

**European Patent Office**

**Office européen des brevets**

(11) **EP 2 719 348 A1**

(12) **EUROPÄISCHE PATENTANMELDUNG**

(43) Veröffentlichungstag:
**16.04.2014 Patentblatt 2014/16**

(51) Int Cl.:
*A61B 17/80* *(2006.01)*

(21) Anmeldenummer: **12007011.5**

(22) Anmeldetag: **10.10.2012**

| | |
|---|---|
| (84) Benannte Vertragsstaaten:<br>**AL AT BE BG CH CY CZ DE DK EE ES FI FR GB GR HR HU IE IS IT LI LT LU LV MC MK MT NL NO PL PT RO RS SE SI SK SM TR**<br>Benannte Erstreckungsstaaten:<br>**BA ME**<br><br>(71) Anmelder: **FACET-LINK Inc.**<br>**Rockaway NJ 07866 (US)** | (72) Erfinder: **Harm-Iven, Jensen**<br>**24214 Noer (DE)**<br><br>(74) Vertreter: **Glawe, Delfs, Moll**<br>**Partnerschaft mbB von**<br>**Patent- und Rechtsanwälten**<br>**Rothenbaumchaussee 58**<br>**20148 Hamburg (DE)** |

(54) **Überdrehgesicherte Knochenschrauben-Fixationsanordnung**

(57)    Eine Fixationsanordnung umfasst einen Halter (2) und eine eine Knochenschraube (1) aufnehmende Spannhülse (3), wobei die Knochenschraube, einen Kopf (13) und ein Sekundärgewinde (12) aufweist und schwenkbeweglich in der Spannhülse gelagert ist, wobei die Spannhülse an ihrem Außenmantel (30) einen radial abstehenden Vorsprung (34) und in ihrem Innenraum (31) mindestens einen Gang eines zum Sekundärgewinde komplementären Spanngewindes (32) aufweist. Im ungespannten Zustand ist ein Freiweg durch ein Spalt-maß an der Spannhülse gebildet. Ein Vorsprung (34) ist vorgesehen , der formschlüssig in eine Aussparung (24) am Aufnahmesitz (23) eingreift, und die Verdickung (15) konusartig nach hinten aufweitend geformt ist, wobei der Konuswinkel ($\beta$) so gewählt ist, dass der Freiweg geteilt durch den Tangens des halben Konuswinkels (tan $\beta/2$) dem 0,5 bis 2,5-fachen der Gewindesteigung (h) des Sekundärgewindes entspricht. Mit dem so gewählten Konuswinkel verschwindet das Spaltmaß nach Anziehen um einen vorbestimmten Anzugswinkel.

Fig. 6

Printed by Jouve, 75001 PARIS (FR)

**Beschreibung**

Die Erfindung betrifft eine Fixationsanordnung umfassend einen Halter ohne Knochenschraube zur Befestigung. Die Fixationsanordnung dient insbesondere zur Verbindung eines Implantats mit umgebendem Knochenmaterial.

Es ist bekannt, zur hinreichend festen Befestigung von Implantaten oder zur Immobilisierung von Gelenken Knochenschrauben zu verwenden. Je nach Anwendungsfall ist hierbei eine winkelstarre Positionierung zwischen dem Implantat und der Knochenschraube ausreichend, in anderen Fällen ist aber eine winkelvariable (polyaxiale) Anordnung erforderlich. Zur Schaffung einer solchen Winkelverstellmöglichkeit ist es bekannt, zwischen dem eigentlich zu haltenden Objekt (Knochenplatte oder Implantat) und der Knochenschraube eine kalottenförmige Spannhülse vorzusehen, die in einem entsprechenden Aufnahmesitz in dem Haltekörper kippbar gelagert ist. Zur Befestigung wird die Knochenschraube durch einen Innenraum der kippbaren Spannhülse geführt, wobei das Gewinde der Knochenschraube nicht nur in den unter dem Haltekörper liegenden Knochen eingreift, sondern mit seinem oberen kopfnahen Bereich in ein entsprechendes Gegengewinde an der Innenwandung der Spannhülse greift. Beim Befestigen der Schraube folgt damit nicht nur eine Befestigung an dem Knochen, sondern die Knochenschraube verzwängt auf die Spannhülse gegenüber dem Haltekörper, so dass diese in ihrer Winkellage fixiert wird. Um die zur Verzwängung notwendige Aufspreizwirkung zu erzielen, ist der Schraubenkopf mit Übermaß relativ zur Weite des Innenraums ausgeführt. Eine derartige Befestigungsanordnung ist beschrieben in US 2005/154392 A1.

Eine Schwierigkeit bei der Handhabung einer derartigen Befestigungsanordnung besteht darin, dass dem Chirurgen nur schwerlich ein Gefühl dafür vermittelt wird, wann die Knochenschraube ausreichend fest angezogen ist. Gerade für Operationen an schwer zugänglichen Stellen oder an kleinen und damit empfindlichen Knochenteilen ist es verhältnismäßig unsicher, sich allein auf ein Gefühl für das Anzugsmoment zu verlassen. Die Verwendung eines Drehmomentschlüssels für sich schafft auch keine ausreichende Abhilfe, da das Anzugsmoment je nach Zustand des Gewindes (trocken oder durch Körperflüssigkeiten benetzt) erheblich variieren kann. Zudem wird das Anzugsdrehmoment bei der vorstehend beschriebenen Befestigungsanordnung noch dadurch verfälscht, dass zusätzliche Kraft zum Aufspreizen der Spannhülse aufgebracht werden muss.

Der Erfindung liegt daher die Aufgabe zugrunde, eine Fixationsanordnung gemäß dem vorstehenden Stand der Technik dahingehend zu verbessern, dass sie einfacher mit einer reproduzierbaren Festigkeit angezogen werden kann.

Die erfindungsgemäße Lösung liegt in einer Fixationsanordnung mit den Merkmalen des unabhängigen Anspruchs. Vorteilhafte Weiterbildungen sind Gegenstand der abhängigen Ansprüche.

Bei einer Fixationsanordnung umfassend einen Halter und eine eine Knochenschraube aufnehmende Spannhülse, wobei die Knochenschraube einen Schaft mit einem Gewinde im vorderen Bereich, einem Kopf und einer Verdickung im hinteren Bereich aufweist, an der ein Sekundärgewinde angeordnet ist, und der Halter mit einer Durchgangsöffnung und einem Aufnahmesitz versehen ist, in dem die Spannhülse schwenkbeweglich gelagert ist, die Spannhülse an ihrem Außenmantel einen radial abstehenden Vorsprung und in ihrem Innenraum mindestens einen Gang eines zum Sekundärgewinde komplementären Spanngewindes aufweist, wobei im ungespannten Zustand ein Freiweg gebildet ist durch ein Spaltmaß an der Spannhülse, ist erfindungsgemäß vorgesehen, dass der Vorsprung formschlüssig in eine Aussparung im Aufnahmesitz eingreift, und die Verdickung konusartig nach hinten aufweitend geformt ist, wobei der Konuswinkel β so gewählt ist, dass der Freiweg geteilt durch den Tangens des halben Konuswinkels (tan β/2) dem 0,5 bis 2,5-fachen der Gewindesteigung des Sekundärgewindes entspricht.

Nachfolgend seien einige verwendete Begriffe erläutert:

Unter "formschlüssig" wird verstanden, dass der Vorsprung derart in der Aussparung geführt ist, dass die Spannhülse bidirektional, d.h. in gegenüberliegende Drehrichtungen, gegenüber einer Verdrehung zum Aufnahmesitz gesichert ist. Dies bedeutet, dass der Vorsprung in dem Aufnahmesitz abgesehen von einem zur Montage erforderlichen geringfügigen Spiel spielfrei gehaltert ist.

In Bezug auf die Knochenschraube wird unter "vorne" die Richtung zu ihrer Spitze am Ende des Schafts und unter "hinten" die gegenüberliegende Richtung zum Kopf der Knochenschraube hin verstanden.

Unter "schwenkbeweglich" wird verstanden, dass die Achse der mittels der Spannhülse fixierten Knochenschraube einen frei einstellbaren Winkel bezogen auf eine Mittelachse der Durchgangsöffnung in der Haltung einnehmen kann. Der Einstellbereich beträgt vorzugsweise mindestens 15° in jeder Richtung und in Mittelachse der Durchgangsöffnung.

Kern der Erfindung ist der Gedanke, durch eine Kombination zweier Maßnahmen eine präzise Definition des Anzugswegs bis zum Erreichen eines festen Sitzes zu schaffen. Der erste Aspekt dieser Kombination liegt darin, dass die Spannhülse mittels des Vorsprungs drehfest gegenüber dem Halter verankert ist. Weiter weist die Spannhülse im Ausgangszustand (montiert, aber noch nicht festgezogen Knochenschraube) ein Spaltmaß zu ihrem Aufnahmesitz und/oder zu der Knochenschraube auf, wobei dieses als Freiweg bezeichnete Spaltmaß durch die konusförmige Gestaltung der Verdickung der Knochenschraube beim Anziehen der Knochenschraube sukzessive verschwindet. Die Rate, mit der der Freiweg verschwindet, hängt von dem Verdrehwinkel der Knochenschraube und der Steigung des Sekundärgewindes an der Verdickung ab. Hierbei sind der durch die Verdrehung der Knochenschraube erreichte Vorschubweg und der durch die konusförmige Gestaltung der Verdickung erreichte Expansionsweg, soweit er schließlich zum Verschwinden des Spaltes führt, nicht identisch, sondern stehen in Bezug zueinander über den Konuswinkel β. Die Erfindung hat erkannt, dass indem dieser Konuswinkel nicht arbiträr, sondern auf eine bestimmte Weise gewählt wird, eine Definition des Anzugswinkels der Schraube bis zum Verschwinden des Freiwegs erreicht werden kann, wenn - und das ist der entscheidende zweite Aspekt der Erfindung - die Spannhülse selbst bidirektional drehfest im Aufnahmesitz gehaltert ist. Denn ohne die bidirektionale, drehfeste Halterung wäre kein festes Bezugssystem gegeben, so dass der Anzugswinkel nicht sinnvoll angegeben werden kann, wenn sich nämlich die Spannhülse mitdreht. Da im praktischen Einsatz es nicht auszuschließen ist, dass ein Chirurg eine Schraube auch einmal zurückdreht, beispielsweise um einen Schraubenschlüssel neu anzusetzen, ist die bidirektionale Festlegung für die praktische Bedeutung enorm wichtig. Es ist das Verdienst der Erfindung, erkannt zu haben, dass die Bestimmung des Konuswinkels in der vorgeschriebenen Weise zwingend kombiniert werden muss mit einer bidirektional drehfesten Fixierung der Spannhülse, um eine praktisch sichere Handhabung und damit Praxistauglichkeit der Fixationsanordnung zu erreichen.

Das den Freiweg bildende Spaltmaß kann auf verschiedene Weise gebildet sein. Zweckmäßigerweise handelt es sich um ein Spiel zwischen der Spannhülse und dem Aufnahmesitz, sozusagen ein äußeres Spiel der Spannhülse. Zusätzlich oder alternativ kann das Freimaß auch gebildet sein durch ein Untermaß der Verdickung der Knochenschraube in Bezug auf die Weite des Innenraums der Spannhülse, also ein inneres Spiel. Beide können auch kombiniert sein. Damit können praxistaugliche, verhältnismäßig große Anzugswinkel von vorzugsweise mindestens 270° entsprechend einer ¾-Umdrehung erreicht werden.

Weiter können im Freiweg auch Elastitzitäten der Knochenschraube, des Halters und insbesondere der Spannhülse berücksichtigt werden. Ist beispielsweise das für die Knochenschraube verwendete Material so elastische, dass sich die Verdickung unter Last komprimiert, so wäre die Kompressionsstrecke zusätzlich bei dem Freiweg zu berücksichtigen. Unbedingt erforderlich ist dies aber nicht, da für viele praktische Anwendungsfälle die verwendeten Materialien als hinreichend steif angesehen werden können.

Zweckmäßigerweise sind die Verdickung und der Kopf der Schraube miteinander kombiniert. Dies ermöglicht eine konstruktiv einfache Gestaltung der Knochenschraube. Zwingend ist die Kombination jedoch nicht.

Besonders bewährt hat sich die Erfindung bei Ausführungsformen, bei denen das Sekundärgewinde und das Gewinde am Schaft der Knochenschraube steigungsverschieden sind. Das Gewinde der Knochenschraube ist in der Regel verhältnismäßig grob (Steigung im Bereich von 1,25 bis 2,5 mm), während für das Sekundärgewinde eher ein Feingewinde in Betracht kommt. Die beiden Gewinde können auf diese Weise dank der Steigungsverschiedenheit an ihre jeweiligen Anwendungszwecke, nämlich Verankerung im Knochenmaterial einerseits bzw. Zusammenwirken mit der meist aus metallischem Material gefertigten Spannhülse andererseits, optimiert werden. Vorzugsweise sind die Steigungen der Gewinde so gewählt, dass das Sekundärgewinde eine kleinere Steigung aufweist als die Steigung des Gewindes am Schaft der Knochenschraube, und zwar vorzugsweise um das 0,4 bis 0,7-fache. Durch die Steigungsverschiedenheit werden beim Anziehen der Knochenschraube die Fragmente, welche durch die Knochenschraube verbunden werden, gegeneinander gezogen. Aus der erfindungsgemäßen Begrenzung des Anzugswinkels resultiert somit eine definierte Verspannung der beiden Knochenfragmente. Der Gefahr eines Überspannens mit dem Risiko eines Ausreißens der Knochenschraube aus dem Gewinde wird damit erfolgreich entgegengewirkt.

Zweckmäßigerweise ist die Höhe des Spanngewindes im Innenraum der Spannhülse beschränkt auf vorzugsweise nicht mehr als drei Gewindegänge, weiter vorzugsweise nicht mehr als anderthalb Gewindegänge. Mit einer solchen Gestaltung ist es besonders einfach, das vordere Gewinde der Knochenschraube durch das Gewinde am Innenmantel des Innenraums der Spannhülse zu führen, und zwar auch dann, wenn es zwar eine unterschiedliche Steigung aber denselben Durchmesser wie das Spanngewinde aufweist.

Die Spannhülse ist zweckmäßigerweise so ausgeführt, dass ihr Innenraum zylindrisch ist. Dies vereinfacht nicht nur die Fertigung, sondern bietet auch den Vorteil, dass der für die Berechnung des Anzugswegs bestimmende Konuswinkel allein durch die Verdickung der Knochenschraube bestimmt ist. Er soll andererseits aber auch nicht ausgeschlossen sein, dass der Innenraum konisch gestaltet ist und im Gegenzug der Außenmantel der Verdickung zylindrisch ausgeführt ist.

Mit Vorteil sind Spannhülse und Aufnahmesitz derart geformt, dass eine Grenzfläche zwischen ihnen kugelartig ist. Besonders zweckmäßig ist, wenn ein Außenmantel der Spannhülse sphärisch geformt ist. Damit ergibt sich eine besonders günstige Gestaltung für einen großen Schwenkwinkel der Spannhülse in ihrem Aufnahmesitz.

Die Spannhülse ist vorzugsweise einfach geschlitzt. Dies bedeutet, dass ein über die gesamte Höhe der Spannhülse verlaufender, durchgehender Schlitz vorgesehen ist. Damit ergibt sich eine gute Aufweitbarkeit, aber auch bessere Komprimierbarkeit zur Vereinfachung der Montage.

Mit Vorteil ist der Vorsprung an der Spannhülse im Bereich der größten Weite (Äquator) der Spannhülse angeordnet. Dies ergibt günstige Verkippeigenschaften unabhängig von der Richtung des Kippwinkels. Weiterhin ist der Vorsprung zweckmäßigerweise rotationssymmetrisch ausgeführt. Dies begünstigt die Gleichmäßigkeit der Verkippeigenschaften unabhängig von der Richtung des Kippwinkels. Ferner bietet diese Gestaltung des Vorsprungs den Vorzug, dass Taumelbewegungen der Spannhülse in ihrem Aufnahmesitz bei der Bewegung vermieden werden. Besonders zweckmäßig hierfür ist eine domförmige, insbesondere halbkugelige Form der Spitze des Vorsprungs. Der Vorsprung kann einen säulenartigen, insbesondere zylindrischen Unterbau aufweisen. Jedoch sollte dieser von der Höhe vorzugsweise gering sein (weniger als die Weite des Vorsprungs), um der Gefahr von Taumelbewegungen entgegenzuwirken.

Besonders zweckmäßig ist es, die im Vorsprung aufnehmende Aussparung im Aufnahmesitz schlitzartig auszuführen, wobei sich die schlitzartige Aussparung in Richtung der Mittelachse der Durchgangsöffnung erstreckt. Damit kann die Spannhülse auf einfache Weise durch Einschieben in ihre Position im Aufnahmesitz montiert werden. Gleichzeitig ist sie dort praktisch spielfrei formschlüssig gehalten. Auf diese Weise werden einfache Montierbarkeit mit sicherer, bidirektional drehfester Halterung kombiniert.

Besonders bewährt hat es sich, einen zweiten Vorsprung diametral gegenüberliegend an der Spannhülse anzuordnen. Es versteht sich, dass dazu eine entsprechende zweite Aussparung am Aufnahmesitz vorzusehen ist. Dies ergibt eine besonders sichere Halterung der Spannhülse, wobei durch die beiden Vorsprünge die Kippachse der Spannhülse exakt definiert ist.

Die schlitzartige Form der Aussparung bietet weiter den Vorteil, dass der Vorsprung sich darin auf und ab bewegen, so dass eine zweite Kippbewegung um eine Achs orthogonal zu der vorgenannten Kippachse ermöglicht ist. Die Bewegungsfreiheit der Spannhülse und damit die Polyaxialität der Befestigung der Knochenschraube ist damit erhöht.

Die Erfindung wird nachfolgend unter Bezugnahme auf die beigefügten Zeichnungen näher erläutert, in denen ein vorteilhaftes Ausführungsbeispiel dargestellt ist. Es zeigen:

Fig. 1 eine Übersichtsdarstellung der Fixationsanordnung gemäß einem Ausführungsbeispiel der Erfindung im montierten Zustand;

Fig. 2 einen teilvergrößerten Ausschnitt aus Fig. 1;

Fig. 3 eine Schnittansicht einer Spannhülse;

Fig. 4 eine Schnittansicht einer Knochenschraube;

Fig. 5a, b schematische Darstellungen zum Freiweg und Konuswinkel;

Fig. 6 eine Montagesicht mit Darstellung des Anzugswinkels; und

Fig. 7 eine alternative Ausführungsform der Knochenschraube.

Eine erfindungsgemäße Fixationsanordnung umfasst eine Knochenschraube 1, einen Halter 2 sowie eine Spannhülse 3. Das in den Figuren dargestellt Ausführungsbeispiel stellt einen Doppelhalter dar, der zwei Knochenschrauben 1 sowie zwei Spannhülsen 3 umfasst.

Eine derartige Fixationsanordnung mit zwei Knochenschrauben 1 kann beispielsweise ausgeführt sein als ein außenbrückendes Implantat zur Verstärkung einer Lamina eines Wirbels (nicht dargestellt), insbesondere nach einer teilweisen Resektion der Lamina. Ein solches Implantat ist Gegenstand einer weiteren Anmeldung derselben Anmelderin mit dem internationalen Anmeldeaktenzeichen PCT/EP2012/001357.

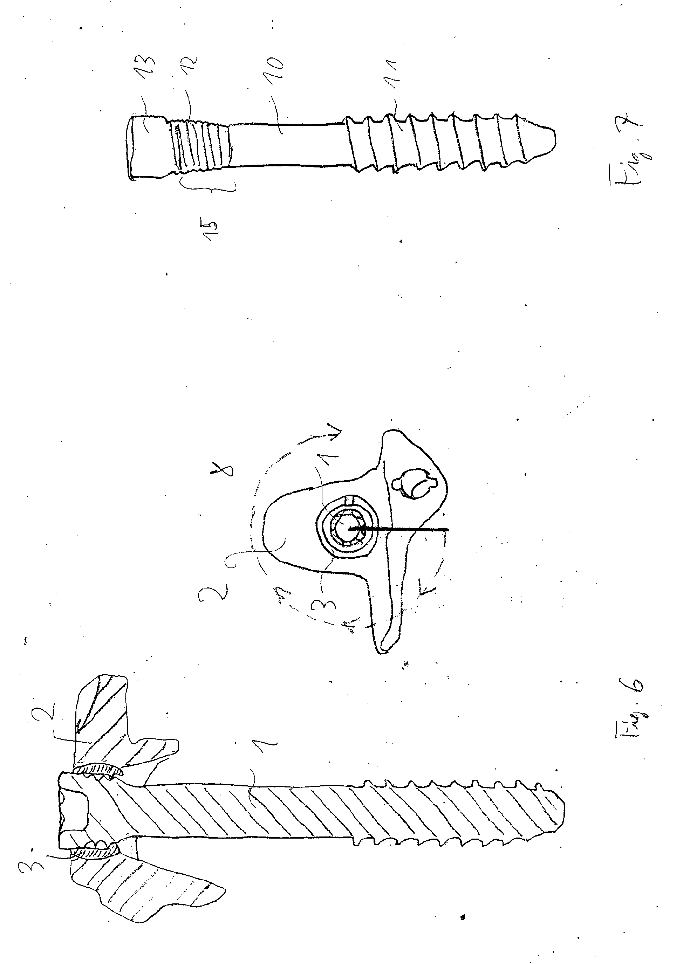

Die Knochenschraube 1 ist an sich herkömmlich ausgeführt mit einem Schaft 10, in dessen vorderen Bereich ein Knochengewinde 11 vorgesehen ist. Das Knochengewinde 11 kann sich auch über den gesamten Schaft 10 erstrecken. Am hinteren Ende des Schafts 10 ist ein als Verdickung ausgeführter Schraubenkopf 13 vorgesehen. Er ist an seiner äußeren Stirnseite mit einer sternförmigen Vertiefung zur Aufnahme eines entsprechend ausgeführten Schraubenschlüssels (nicht dargestellt) versehen. Der Schraubenkopf 13 ist konusförmig gestaltet und weist an seinem Außenmantel 14 einen Konuswinkel $\beta$ auf. An dem Außenmantel 14 ist ein Sekundärgewinde 12 angeordnet. Es ist gleichsinnig zu dem Knochengewinde 11 ausgeführt, weist jedoch eine beträchtlich kleinere Steigung h auf (in dem dargestellten Ausführungsbeispiel nur die Hälfte der Steigung H des Knochengewindes 11). Das Sekundärgewinde 12 erstreckt sich etwa über drei Gewindegänge. Die Verdickung 15 kann auch gesondert von dem Schraubenkopf 13 ausgeführt sein (s. Fig. 7).

Der Halter 2 weist an seinen beiden Enden je eine Durchgangsöffnung 20 zur Aufnahme einer der Knochenschrauben 1 auf. Sie ist in ihrem mittleren Bereich kugelkalottenförmig erweitert und bildet somit einen Aufnahmesitz 23 für die Spannhülse 3. An einer Seite des Aufnahmesitzes 23 ist eine schlitzartige Aussparung 24 ausgeformt, die sich mindestens über die halbe Tiefe der Durchgangsöffnung 20 erstreckt. Der Aufnahmesitz 23 dient zur schwenkbeweglichen Halterung der Spannhülse 3. Dazu weist die Spannhülse 3 einen sphärischen Außenmantel 30 auf, dessen Kontur im Wesentlichen komplementär ist zu derjenigen des Aufnahmesitzes 23. Die Spannhülse 3 weist eine zentrale Öffnung auf, die einen zylindrischen Innenraum 31 bildet. Durch diesen ist im montierten Zustand die Knochenschraube 1 gesteckt. Dabei kämmt die Knochenschraube 1 mit einem anderthalbfachen Gewindegang eines Spanngewindes 32, welches an der Wandung des Innenraums 31 angeordnet ist und komplementär ist zum Sekundärgewinde 12 der Knochenschraube 1.

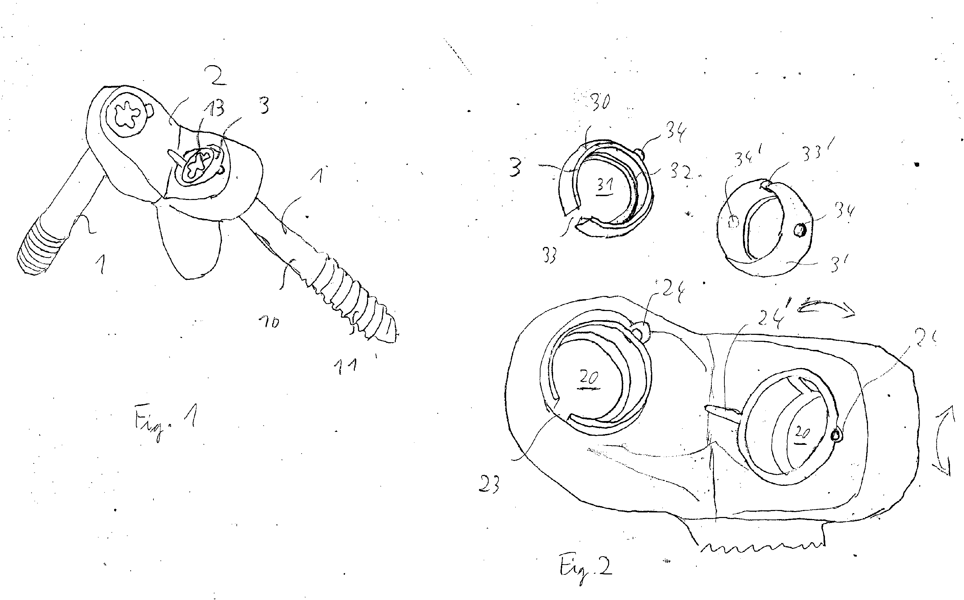

Die Spannhülse 3 weist weiter an einer Seite einen durchgehenden Schlitz 33 auf. Gegenüberliegend ist an dem Außenmantel 31 der Spannhülse 3 ein radial nach außen abragender Vorsprung 34 am Äquator 35 angeordnet. Der Vorsprung 34 ist in dem dargestellten Ausführungsbeispiel halbkugelförmig geformt. Seine Abmessungen sind passend zu denen der schlitzartigen Aussparung 24 gewählt, so dass der Vorsprung 34 mit geringfügigem Spiel (unter geringfügig wird im Sinne dieser Anmeldung etwa ein Spiel von 5% bis 30% der Weite des Vorsprungs verstanden) formschlüssig in der schlitzartigen Aussparung 24 einschiebbar ist. Im montierten Zustand ist so die Spannhülse 3 bidirektional vor einer Verdrehung relativ zum Halter 2 gesichert. Die halbkugelförmige Gestaltung des Vorsprungs 34 erlaubt in Verbindung mit der schlitzartigen Aussparung 24 eine doppelkardanische Lagerung der Spannhülse 3 in dem Halter 2, wie sie in Fig. 2 durch die beiden Pfeile in der rechten Bildhälfte visualisiert ist. Damit ist eine achsvariable (polyaxiale) Lagerung der Knochenschraube 1 in dem Halter 2 erreicht. Praktisch wird so ein Winkelverstellbereich von ca. 15 Grad in jede Richtung ermöglicht.

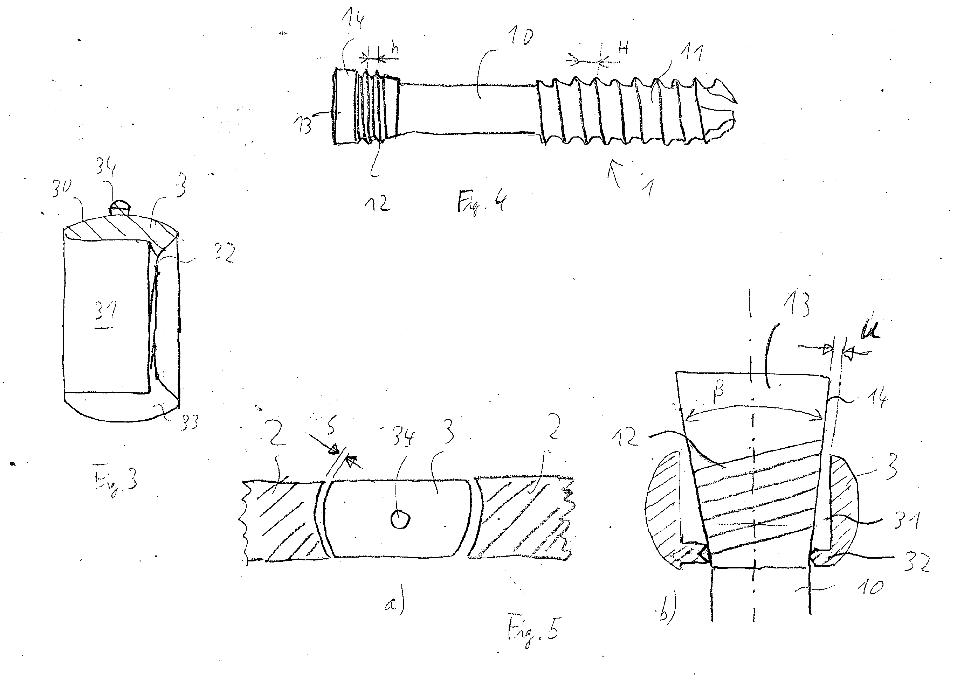

Es wird nun Bezug genommen auf die Schnittdarstellungen in Fig. 5a und 5b. Aus praktischen Gründen einer leichten Montierbarkeit und besserer Schwenkbeweglichkeit sitzt die Spannhülse 3 im Ausgangszustand mit Spiel in ihrem Aufnahmesitz 23. Das bedeutet, dass der Innendurchmesser des kugelkalottenförmigen Aufnahmesitzes 23 größer ist als der Außendurchmesser des sphärischen Außenmantels 30 der Spannhülse 3.

Weiter weist in dem dargestellten Ausführungsbeispiel die Knochenschraube 1 im Bereich der konusförmigen Verdickung, hier dem Schraubenkopf 13, ein Untermaß U auf. Das Untermaß U ist definiert als der Spalt zwischen Konusmantel 14 und dem Rand des Innenraums 31 in dem Zustand, wenn die Knochenschraube 1 mit ihrem Sekundärgewinde 12 das Spanngewinde 32 erfasst (s. Fig. 5b).

Somit ergibt sich insgesamt durch das Spiel S und das Untermass U ein Freiweg F, der durch die Summe des Spiel S und des Untermass U bestimmt ist gemäß der Beziehung

$$F = S + U.$$

Dieser Freiweg F wird beim Festziehen der Knochenschraube 1 aufgrund der konischen Aufweitung der Schraubenkopfs 13 allmählich vermindert. Ist es ganz verschwunden, dann ist die Knochenschraube 1 festgezogen. Weiteres Anziehen könnte dann zu einer Überlastung der Schraubverbindung führen, insbesondere kann es zu einem unerwünschten Ausreißen des Knochengewindes 11 aus dem Wirbelkörper kommen.

Um dies sicher zu verhindern wird ein bestimmter Anzugwinkel $\gamma$ definiert, den der Chirurg zum Festziehen anzuwenden hat. Er kann beispielsweise etwa 270 Grad betragen, also eine Dreiviertelumdrehung (s. Fig. 6). Ausgangspunkt hierfür ist die Position, bei der die Knochenschraube mit ihrem Sekundärgewinde 12 gerade in das Spanngewinde 32 eingreift (s. Fig. 5b). Ein solches Maß von 270 Grad (entsprechend einer Dreiviertelumdrehung) stellt einen für die praktische Anwendbarkeit guten Kompromiss aus guter Einstellbarkeit einerseits und verwechselungssicherer Handhabung andererseits dar. Um nun zu erreichen, dass der Freiweg F nach einer Dreiviertelumdrehung aufgezehrt ist, muss der Konuswinkel $\beta$ entsprechend gewählt sein. Erfindungsgemäß erfolgt dies gemäß der Beziehung

$$F / \tan(\beta/2) = 270°/360° * h,$$

wobei h die Steigung des Sekundärgewindes 12 ist. Zur Berechnung des gesuchten Konuswinkel $\beta$ kann die Beziehung umgestellt werden zu

$$\beta = 2 * \arctan(F / (0,75 * h)).$$

Die vorgenannte Berechnungsvorschrift für den Konuswinkel $\beta$ gilt unabhängig davon, ob der Freiweg F durch das Spiel S allein oder das Untermaß U allein oder durch eine Kombination beider gebildet ist.

**EP 2 719 348 A1**

Die Definition des Anzugswinkels, hier im Beispiel 270 Grad für eine Dreiviertelumdrehung, ist jedoch sinnlos, wenn die Spannhülse nicht verdrehgesichert ist. Denn ohne eine Verdrehsicherung kann es zu einem Mitdrehen der Spannhülse kommen, wodurch die Definition des Anzugswinkels ihre Basis verliert. Dieser Gefahr wirkt die Erfindung entgegen, indem sie die Spannhülse 3 mittels des Vorsprungs 34 in der schlitzartigen Aussparung 24 drehfest haltert, und zwar bidirektional. Erst damit wird eine praktische Umsetzbarkeit der Definition eines Anzugswinkels erreicht. Da die Spannhülse 3 drehfest an dem Halter 2 gehalten ist, bleibt der Bezugspunkt für den Anzugswinkel erhalten. Dies gilt sogar dann, wenn es zu einem an sich nicht vorgesehenen, in der Praxis aber eben doch nicht selten vorkommenden Rückdrehen kommt, zum Beispiel beim Umsetzen des Schraubenschlüssels. Erst in der Kombination mit dieser bidirektional wirkenden Halterung der Spannhülse 3 erhält das Konzept des Anzugswinkels praktische Brauchbarkeit in einem Operations-Umfeld.

Ist die Knochenschraube 1 um den bestimmten Anzugswinkel $\gamma$ angezogen, wird eine doppelte Wirkung erreicht. Zum einen ist die Spannhülse 3 sicher verspannt gegen den Halter 2 und die Achse der Knochenschraube ist somit festgelegt. Zum anderen ist die Knochenschraube 1 nun fest genug angezogen für eine sichere Befestigung im Wirbel, aber auch nicht zu fest, so dass es nicht zu einer Beschädigung des Wirbels aufgrund eines wegen Überbeanspruchung ausreißenden Knochengewindes aus dem Wirbel kommen kann. Verbindet die Knochenschraube 1 zwei Knochenfragmente, so werden sie mit einer wohldefinierten Verspannung gegeneinander gezogen.

Es wird also sicheres, festes Anziehen kombiniert mit einem praxistauglichen Überdrehschutz.

Bei einer alternativen Ausführungsform, wie in Fig. 2 in der rechten Bildhälfte dargestellt ist, kann vorgesehen sein, dass zwei Vorsprünge 34, 34' an einer Spannhülse 3' angeordnet sind. Dazu ist ein zweiter Vorsprung 34' vorgesehen, der identisch zu dem Vorsprung 34 ausgeführt ist. Er ist diesen diametral gegenüberliegend an dem Außenmantel der Spannhülse 3 angeordnet, weist also in die entgegengesetzte Richtung. Der Schlitz 33' ist in diesem Fall an einer Stelle zwischen den beiden Vorsprüngen 34, 34' an der Spannhülse 3 angeordnet, vorzugsweise in einer Querposition. An dem Halter 2 ist der Aufnahmesitz 23 mit einer entsprechenden zweiten schlitzartigen Aussparung 24' versehen. Mit den doppelten Vorsprüngen 34, 34' wird eine zweite Kippachse definiert, so dass eine sicher geführte doppelkardanische Beweglichkeit der Spannhülse 3 in dem Halter 2 erreicht wird.

**Patentansprüche**

1. Fixationsanordnung umfassend einen Halter (2) und eine eine Knochenschraube (1) aufnehmende Spannhülse (3), wobei die Knochenschraube (1) einen Schaft (10) mit einem vorderen Gewinde (11), einem Kopf (13) und einer Verdickung (15) mit einem Sekundärgewinde (12) aufweist, der Halter (2) mit einer Durchgangsöffnung (20) und einem Aufnahmesitz (23) versehen ist, in dem die Spannhülse (3) schwenkbeweglich gelagert ist, die Spannhülse (3) an ihrem Außenmantel (30) einen radial abstehenden Vorsprung (34) und in ihrem Innenraum (31) mindestens einen Gang eines zum Sekundärgewinde (12) komplementären Spanngewindes (32) aufweist, wobei im ungespannten Zustand ein Freiweg (F) durch ein Spaltmaß an der Spannhülse (3) gebildet ist, **dadurch gekennzeichnet, dass** der Vorsprung (34) formschlüssig in eine Aussparung (24) am Aufnahmesitz (23) eingreift, und die Verdickung (15) konusartig nach hinten aufweitend geformt ist, wobei der Konuswinkel ($\beta$) so gewählt ist, dass der Freiweg (F) geteilt durch den Tangens des halben Konuswinkels (tan $\beta/2$) dem 0,5 bis 2,5-fachen der Gewindesteigung (h) des Sekundärgewindes (12) entspricht.

2. Fixationsanordnung nach Anspruch 1, **dadurch gekennzeichnet, dass** der Freiweg (F) ein Untermaß (U) umfasst, das gebildet ist durch einen Spalt zwischen Verdickung (15) und dem Innenraum (31) der Spannhülse (3).

3. Fixationsanordnung nach Anspruch 1 oder 2, **dadurch gekennzeichnet, dass** der Freiweg (F) ein Spiel (S) zwischen Spannhülse (3) und dem Aufnahmesitz (23) umfasst.

4. Fixationsanordnung nach einem der vorangehenden Ansprüche, **dadurch gekennzeichnet, dass** die Verdickung (15) und der Kopf (13) kombiniert sind.

5. Fixationsanordnung nach einem der vorangehenden Ansprüche, **dadurch gekennzeichnet, dass** das Sekundärgewinde (12) und das Gewinde (11) am Schaft (10) der Knochenschraube (1) steigungsverschieden sind.

6. Fixationsanordnung nach Anspruch 5, **dadurch gekennzeichnet, dass** das Sekundärgewinde (12) eine kleinere Steigung aufweist, vorzugsweise das 0,4 bis 0,7-fache.

7. Fixationsanordnung nach einem der vorangehenden Ansprüche, **dadurch gekennzeichnet, dass** der Innenraum (31) der Spannhülse (3) zylindrisch ist.

8. Fixationsanordnung nach einem der Ansprüche 1 bis 6, **dadurch gekennzeichnet, dass** der Innenraum der Spannhülse konisch ist und der Außenmantel der Verdickung zylindrisch ist.

9. Fixationsanordnung nach einem der vorangehenden Ansprüche, **dadurch gekennzeichnet, dass** die Höhe des Spanngewindes (32) höchstens das Dreifache, vorzugsweise das Anderthalbfache der Ganghöhe (H) des Gewindes (11) am Schaft (10) der Schraube (1) beträgt.

10. Fixationsanordnung nach einem der vorangehenden Ansprüche, **dadurch gekennzeichnet, dass** eine Grenzfläche zwischen Spannhülse (3) und Aufnahmesitz (23) kugelartig ist, insbesondere der Außenmantel (30) der Spannhülse (3) sphärisch geformt ist.

11. Fixationsanordnung nach einem der vorangehenden Ansprüche, **dadurch gekennzeichnet, dass** die Spannhülse (3) einfach geschlitzt ist.

12. Fixationsanordnung nach einem der vorangehenden Ansprüche, **dadurch gekennzeichnet, dass** der Vorsprung (34) im Bereich der größten Weite der Spannhülse (3), vorzugsweise an deren Äquator (35), angeordnet ist.

13. Fixationsanordnung nach einem der vorangehenden Ansprüche, **dadurch gekennzeichnet, dass** der Vorsprung (34) rotationssymmetrisch ausgeführt ist.

14. Fixationsanordnung nach Anspruch 13, **dadurch gekennzeichnet, dass** der Vorsprung (34) mit einer domförmigen, vorzugsweise halbkugeligen Spitze ausgeführt ist.

15. Fixationsanordnung nach Anspruch 13 oder 14, **dadurch gekennzeichnet, dass** der Vorsprung (34) einen säulenartigen, insbesondere zylinderförmigen Unterbau aufweist.

16. Fixationsanordnung nach einem der vorangehenden Ansprüche, **dadurch gekennzeichnet, dass** die Aussparung (24) im Aufnahmesitz (23) schlitzartig ist und sich in Richtung einer Mittelachse der Durchgangsöffnung (20) erstreckt.

17. Fixationsanordnung nach einem der vorangehenden Ansprüche, **dadurch gekennzeichnet, dass** ein zweiter Vorsprung (34') diametral gegenüberliegend angeordnet ist, wobei im Aufnahmesitz (23) eine entsprechende zweite Aussparung (24') ausgearbeitet ist.

Fig. 2

Fig. 1

14   h   10   H   11

13

12   Fig. 4   1

34
30   3
22
31
33

Fig. 3

2   5   34   3   2

a)

Fig. 5

1   13
u
14
β
12   3
31
32
b)   10

Fig. 7

Fig. 6

**EP 2 719 348 A1**

Europäisches
Patentamt

European
Patent Office

Office européen
des brevets

## EUROPÄISCHER RECHERCHENBERICHT

Nummer der Anmeldung

EP 12 00 7011

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | US 2010/324604 A1 (MATHIEU CLAUDE [CH] ET AL) 23. Dezember 2010 (2010-12-23) * Seite 1, Absatz 11-12 * * Seite 2, Absatz 32 - Seite 3, Absatz 41 * <br> * Abbildungen 3, 6-8 * ----- | 1,3,4,7, 10-17 | INV. A61B17/80 |
| A | US 2008/306550 A1 (MATITYAHU AMIR M [US]) 11. Dezember 2008 (2008-12-11) <br> * Seite 4, Absatz 53 - Seite 5, Absatz 61 * <br> * Abbildungen 10, 18, 21-23 * ----- | 1,3,4,7, 10-13, 15-17 | |
| A | US 2009/062862 A1 (PERROW SCOTT J [US] ET AL) 5. März 2009 (2009-03-05) <br> * Seite 7, Absatz 103 - Seite 8, Absatz 108 * * Abbildungen 14-18D * ----- | 1,7,10, 12,13, 15,17 | |
| A | DE 10 2010 042930 A1 (DIETER MARQUARDT MEDIZINTECHNIK GMBH [DE]) 26. April 2012 (2012-04-26) * Seite 4, Absatz 31 - Seite 5, Absatz 42 * <br> * Anspruch 8 * * Abbildungen 3-11 * ----- | 1,4-6 | RECHERCHIERTE SACHGEBIETE (IPC) <br> A61B |

Der vorliegende Recherchenbericht wurde für alle Patentansprüche erstellt

| Recherchenort | Abschlußdatum der Recherche | Prüfer |
|---|---|---|
| Berlin | 19. März 2013 | Kakoullis, Marios |

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 12 00 7011

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

19-03-2013

| Im Recherchenbericht angeführtes Patentdokument | Datum der Veröffentlichung | Mitglied(er) der Patentfamilie | | Datum der Veröffentlichung |
|---|---|---|---|---|
| US 2010324604 A1 | 23-12-2010 | AR | 037904 A1 | 22-12-2004 |
| | | AT | 306221 T | 15-10-2005 |
| | | AU | 2002220448 A1 | 15-07-2003 |
| | | BR | 0117199 A | 14-12-2004 |
| | | CA | 2471843 A1 | 10-07-2003 |
| | | CN | 1582132 A | 16-02-2005 |
| | | DE | 50107716 D1 | 17-11-2005 |
| | | DK | 1458299 T3 | 20-02-2006 |
| | | EP | 1458299 A1 | 22-09-2004 |
| | | ES | 2250307 T3 | 16-04-2006 |
| | | JP | 4125238 B2 | 30-07-2008 |
| | | JP | 2005512725 A | 12-05-2005 |
| | | MY | 134176 A | 30-11-2007 |
| | | NZ | 533664 A | 28-01-2005 |
| | | SI | 1458299 T1 | 30-04-2006 |
| | | TW | I225396 B | 21-12-2004 |
| | | US | 2005043736 A1 | 24-02-2005 |
| | | US | 2008172094 A1 | 17-07-2008 |
| | | US | 2010137867 A1 | 03-06-2010 |
| | | US | 2010324604 A1 | 23-12-2010 |
| | | US | 2012259371 A1 | 11-10-2012 |
| | | WO | 03055401 A1 | 10-07-2003 |
| US 2008306550 A1 | 11-12-2008 | EP | 2164413 A1 | 24-03-2010 |
| | | US | 2008306550 A1 | 11-12-2008 |
| | | WO | 2008154050 A1 | 18-12-2008 |
| US 2009062862 A1 | 05-03-2009 | KEINE | | |
| DE 102010042930 A1 | 26-04-2012 | KEINE | | |

Für nähere Einzelheiten zu diesem Anhang : siehe Amtsblatt des Europäischen Patentamts, Nr.12/82

EPO FORM P0461

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2005154392 A1

- EP 2012001357 W